# EUROPEAN PATENT APPLICATION

(11) **EP 0 922 437 A1**
(43) Date of publication of application: **16.06.1999**
(21) Application number: 98122146.8
(22) Date of filing: 25.11.1998
(51) Int. Cl.: A61B 17/72

(54) **Intramedullary nail for the osteosynthesis of bone fractures**

(30) Priority: 11.12.1997 IT MI972744
(71) Applicant: ORTOMEDICAL S.p.A., 24060 Cividino di Castelli di Calepio (Bergamo) (IT)
(72) Inventor: Monfardini, Ennio Alessio, 25036 Palazzolo S/O (Brescia) (IT)
(74) Representative: Modiano, Guido, Dr.-Ing.

(57) **Abstract**

An intramedullary nail for use in osteosynthesis for the surgical treatment of diaphyseal and metaphyseal fractures of the femur and tibia, comprising a tube (2) which is laterally provided, in its proximal (3) and distal (4) regions, with holes (5,6) for the insertion of at least one pair of wire rods which are pointed at one end (7) and are provided with a protruding head (8) at the other end; the holes (5,6) are formed at an angle with respect to the longitudinal axis of the tube and the wire rods are angled at their distal portion that is suitable to enter the angled holes.

## Description

The present invention relates to an intramedullary nail for osteosynthesis for the surgical treatment of diaphyseal and metaphyseal fractures of the femur and tibia.

Surgical treatment of diaphyseal and metaphyseal fractures of the femur and tibia is conventionally performed by means of an intramedullary nail which is inserted in a hole formed in the femur or tibia.

The intramedullary nail is provided with an internal channel for the passage of a pair of wire rods which protrude from a distal end of the nail through holes formed on its lateral surface in order to lock the intramedullary nail within the femur or tibia in which it is arranged.

The wire rods inserted in the intramedullary nail are pointed at one end and have a protruding head at their other end.

One drawback of the intramedullary nail thus provided is the fact that the openings provided in the distal end of the intramedullary nail, on its lateral surface, must be crossed by the pointed ends of the wire rods: this is very difficult, since the wire rods inserted in the nail do not tend to spontaneously pass through the lateral holes of the intramedullary nail but rather tend to exit from the distal end of said nail, which is open.

Accordingly, this entails difficult positioning of the wire rods inside the intramedullary nail, also in view of the difficulty that the surgeon encounters in the insertion of said wire rods while the nail is inserted in the femur or tibia of the patient.

The wire rods that are currently inserted in the intramedullary nail are kept in position by a threaded nut which is screwed at the threaded proximal end of the intramedullary nail.

The insertion of the nut also entails difficulties, since it can be inserted incorrectly and therefore the threads of the nut and of the internal surface of the intramedullary nail do not mesh perfectly, consequently jamming the nut. Any damage to the thread of the intramedullary nail entails not only difficulty in screwing the nut within the nail but also subsequent difficulty in extracting the intramedullary nail when the surgeon must remove it from the femur or tibia.

Moreover, the implantation of the intramedullary nail, performed without the surgeon being able to directly view the operation that he is performing, entails the use of X-rays, with consequent danger for the person performing the implantation and for the patient.

This occurs in particular when the surgeon must fix the intramedullary nail to the bone (femur or tibia) by means of screws provided in addition to the wire rods for distal and proximal fixing of the nail.

The aim of the present invention is to provide an intramedullary nail for use in osteosynthesis for the surgical treatment of diaphyseal and metaphyseal fractures of the femur and tibia in which insertion of the wire rods in distal holes of the intramedullary nail is facilitated.

Within the scope of this aim, an object of the present invention is to provide an intramedullary nail in which means are provided for facilitating the insertion of the locking plug (nut) of the wire rods.

Another object of the present invention is to provide an intramedullary nail which allows to avoid all distal locking manoeuvres performed by means of screws, which require considerable time and the use of X-rays which are harmful for the surgeon and for the patient.

Another object of the present invention is to provide an intramedullary nail in which the insertion of the locking screw at the proximal end of the nail is performed without resorting to X-rays.

Another object of the present invention is to provide an intramedullary nail which is highly reliable, relatively easy to manufacture and at competitive costs.

This aim, these objects and others which will become apparent hereinafter are achieved by an intramedullary nail for use in osteosynthesis for the surgical treatment of diaphyseal and metaphyseal fractures of the femur and tibia, comprising a tube which is laterally provided, in proximal and distal regions thereof, with holes for the insertion of at least one pair of wire rods which are pointed at one end and are provided with a protruding head at the other end, characterized in that said holes are formed at an angle with respect to a longitudinal axis of said tube, said wire rods being angled at their distal portion that enters said angled holes.

Further characteristics and advantages of the present invention will become apparent from the following detailed description of a preferred but not exclusive embodiment of the intramedullary nail according to the invention, illustrated only by way of non-limitative example in the accompanying drawings, wherein:

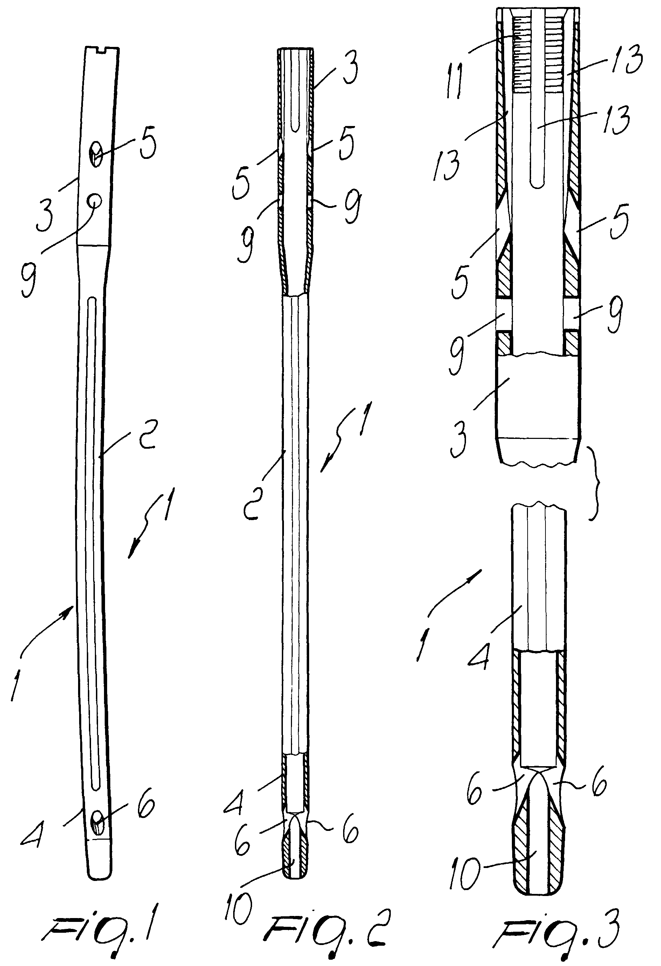

Figure 1 is a view of an intramedullary nail according to the present invention;
Figure 2 is a view of the intramedullary nail of Figure 1, with the proximal and distal parts shown in cross-section;
Figure 3 is a longitudinal sectional view of the proximal and distal ends of the intramedullary nail according to the invention;

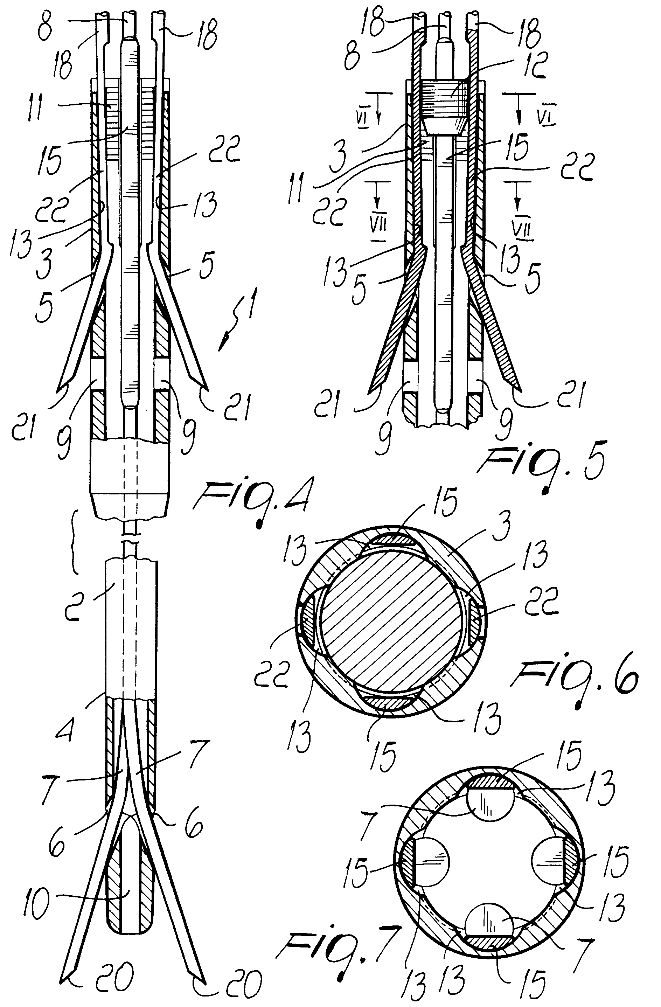

Figure 4 is a view of the proximal and distal ends of the intramedullary nail according to the invention, with the wire rods inserted in the nail;
Figure 5 is a partial sectional view of the proximal end of the intramedullary nail according to the invention;

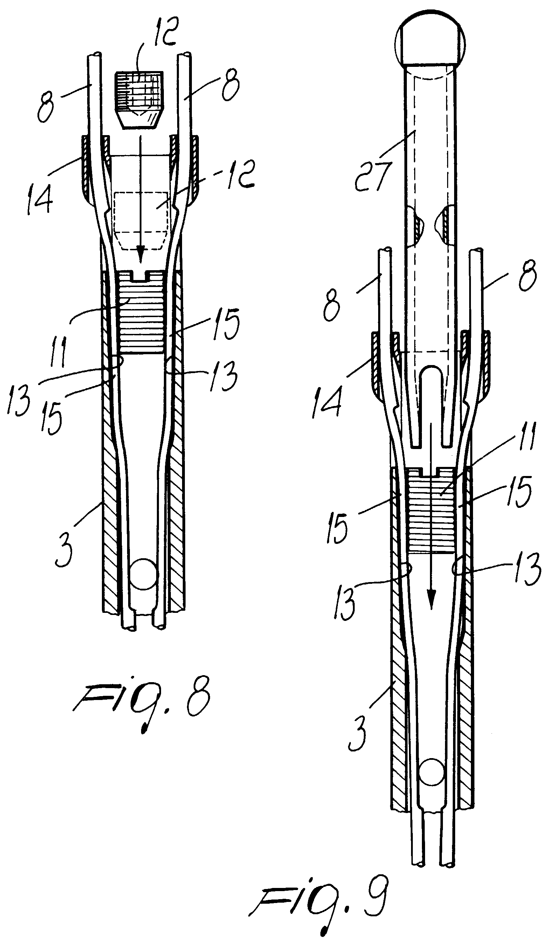

Figure 6 is a sectional view of the intramedullary nail according to the invention, taken along the plane VI-VI of Figure 5;
Figure 7 is a sectional view of the intramedullary nail according to the invention, taken along the plane VII-VII of Figure 5;
Figure 8 is a sectional view of the proximal end of the intramedullary nail according to the invention, illustrating the insertion of the threaded plug;

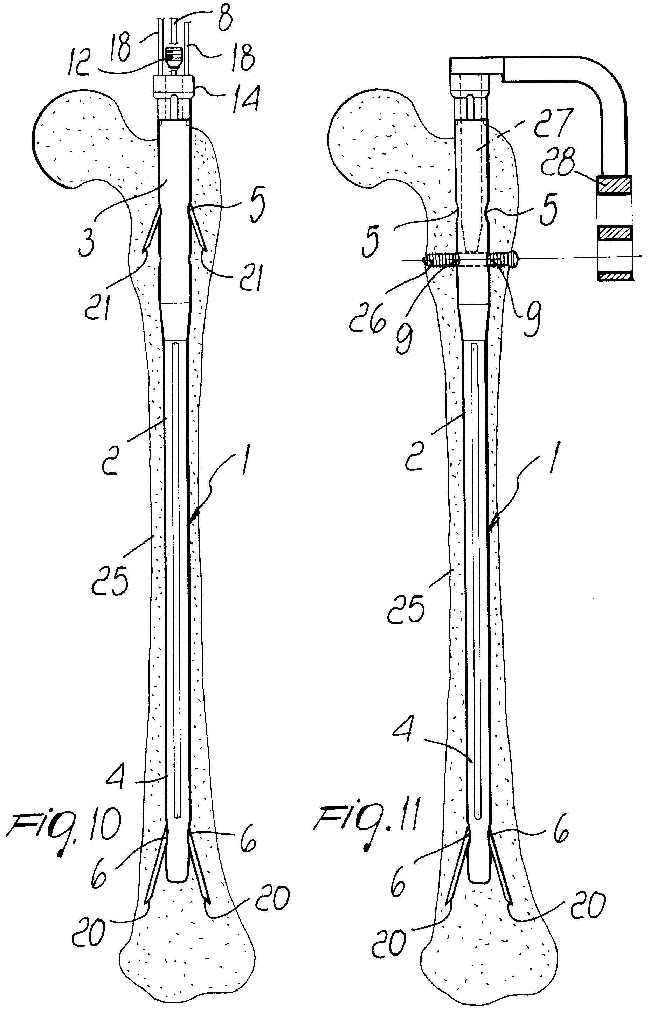

Figure 9 is a sectional view of the proximal portion of the intramedullary nail according to the invention, illustrating the insertion of a tool for determining the insertion point of the fixing screws;
Figure 10 is an exemplifying view of a femoral bone with an intramedullary nail according to the present invention inserted therein; and
Figure 11 is a view of a femoral bone with an intramedullary nail according to the present invention inserted therein, also illustrating the tool for determining the screw insertion point shown in Figure 9.

With reference to the above figures, the intramedullary nail according to the present invention, generally designated by the reference numeral 1, comprises a tube 2 which acts as a stem and is laterally provided, in its proximal region 3 and distal region 4, with passages or holes, designated by the reference numerals 5 and 6 respectively, for the insertion of at least one pair of wire rods which have points at one end, designated by the reference numeral 7, and are provided with a protruding head, designated by the reference numeral 8, at the other end.

Each one of the holes 5 is conveniently formed at an angle which is suitable to facilitate the insertion of another pair of wire rods 18, whose function will be explained in detail hereinafter.

Likewise, each one of the holes 6 is conveniently formed at an angle which allows easier insertion of the wire rods 7 for their exit from the lateral surface of the intramedullary nail 2.

Each one of the wire rods 7 is constituted by a tubular portion which blends into a flat central portion 15 and then continues with a tubular portion which bends, at the distal end of the wire rod 7, at an angle with respect to the overlying portions of the wire rod.

The point 20 of each wire rod is chamfered so as to allow insertion in the angled holes 6; likewise, the point 21 of each one of the wire rods 18 is also chamfered so as to enter the angled holes 5.

The pairs of holes 5 and 6 are therefore angled with respect to the diametrical axis of the intramedullary nail 2.

The intramedullary nail 2 has, at its distal end, an opening 10 which is used to position the nail inside the femur or tibia of a patient.

Each one of the wire rods 7 and 18 is formed by a wire whose diameter can vary according to requirements, and the wire rods 7 have an angled point 20 which is preferably rotated through 90° with respect to the flat central portion 15.

The wire rods 18 instead have a point 21 which is angled with respect to their flat central portion 22 but is not rotated through 90°.

The tube 2 of the intramedullary nail 1 has, at its proximal end 3, a threaded portion 11 which is meant to accommodate a substantially conical plug 12 which is also threaded and is meant to lock the wire rods 18 and 7 against the internal lateral walls of the tubular body 2 of the intramedullary nail 1.

For this purpose, the tubular body 2 of the intramedullary nail is internally provided, along its internal circumference, with a number of grooves 13 which is equal to the number of wire rods inserted therein.

A portion of the wire rods 18 and 7 enters each one of the grooves 13 and is hemispherically shaped so as to fit inside the grooves 13 and subsequently not interfere with the insertion of the locking plug 12, which is screwed on the thread 11 so as to lock by interference the wire rods 18 and 7 against the wall of the tubular body 2 of the intramedullary nail, in order to divaricate the wire rods 18 and 7 so as to allow them to exit fully from the holes 5 and 6.

Advantageously, the angle of the holes 5 and 6 has been found to be optimum when it reaches approximately 25° with respect to the longitudinal axis of the tubular body 2; however, other wider or smaller angles can also be used advantageously.

Holes 9 are used to lock the intramedullary nail 1 within the femoral body, shown in detail in Figures 10 and 11 and designated by the reference numeral 25, if one does not wish to use the wire rods 18 that protrude from the holes 5.

Use of the intramedullary nail is of course also valid for the tibia of a patient; in this case, the tubular body 2, instead of being straight as shown in the figures, has a straight portion followed by a portion which is slightly angled with respect to said straight portion.

As mentioned, the holes 9 are meant to lock, by means of a screw 26, the intramedullary nail 1 to the femoral body 25.

In order to determine the correct point for forming the hole for inserting the screw 26, which must therefore pass through the pair of holes 9, a tool designated by the reference numeral 27 is advantageously used. The tool is provided with a point which enters the cavity of the tubular body 2, and its other end is shaped so as to have a sort of template 28 which allows to determine the position in which the hole for the insertion of the screw 26 is to be formed.

In order to provide correct insertion of the threaded plug 12 so as to engage the thread 11 of the tubular body 2, a bush 14 is used which is arranged at the proximal end of the intramedullary nail 1 and through which the wire rods 18 and 7 are inserted.

The bush 14 is also used to insert the tool or instrument 27 when the wire rods 18 are not used and the nail 1 is instead fixed to the bone by means of the screw 26.

The grooves 13 have a substantially conical profile which tapers from the top, i.e., from the proximal portion of the tubular body 2, in a downward direction, so that the thickness of the wire rods that rest on the bottom of the grooves 13 becomes such as to interfere with the ridge of the threaded plug 12.

The grooves 13 therefore substantially affect the proximal end 3 of the tubular body 2 of the intramedullary nail.

The particular point 20 and 21 of the wire rods 18 and 7 allows the wire rods to engage the nearest cortical bone tissue, locking the intramedullary nail to the walls of the bone.

It is thus advantageously possible to avoid all manoeuvres for distal locking by means of screws, which require considerable time and have a high degree of uncertainty since centering of the distal holes 6 is often difficult and also requires a considerable use of X-rays which are harmful for the surgeon and the patient.

The same occurs for the proximal locking of the intramedullary nail by means of the wire rods 18 as an alternative to conventional screws for cortical bone.

In practice it has been observed that the intramedullary nail according to the invention fully achieves the intended aim and objects, since it allows to lock the wire rods in position without using screws or distal locking means but most of all allows the wire rods to easily enter the respective distal holes or the proximal holes for the upper locking wire rods, allowing the surgeon to operate precisely and accordingly reducing the time required for operating.

Since the instrument used to determine the drilling points for the insertion of the upper locking screws is provided with one or more external holes and with a point which is meant to enter the tubular body of the intramedullary nail, it allows to determine the insertion point of the locking screw without having to perform an approximate measurement as currently occurs.

The intramedullary nail thus conceived is susceptible of numerous modifications and variations, all of which are within the scope of the inventive concept; all the details may also be replaced with other technically equivalent elements.

The materials employed, as well as the dimensions, may be any according to requirements and to the state of the art.

The disclosures in Italian Patent Application No. MI97A02744 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An intramedullary nail for use in osteosynthesis for the surgical treatment of diaphyseal and metaphyseal fractures of the femur and tibia, comprising a tube which is laterally provided, in proximal and distal regions thereof, with holes for the insertion of at least one pair of wire rods which are pointed at one end and are provided with a protruding head at the other end, characterized in that said holes are formed at an angle with respect to a longitudinal axis of said tube, said wire rods being angled at their distal portion that enters said angled holes.

2. The intramedullary nail according to claim 1, characterized in that said wire rods have a first cylindrical portion which is followed by a substantially flat central portion and by a cylindrical end portion.

3. The intramedullary nail according to claim 2, characterized in that a proximal region of said tube is internally provided with a number of grooves which is equal to the number of said wire rods, said grooves being conical and tapering from the top downward in order to accommodate the substantially flat central portion of said wire rods.

4. The intramedullary nail according to claim 1, characterized in that it comprises a first pair of wire rods for the distal locking of said tube and a second pair of wire rods for a proximal locking of said tube.

5. The intramedullary nail according to claim 3, characterized in that said proximal region of the tube comprises an internally threaded portion which is suitable to accommodate a likewise threaded plug for spacing and locking said wire rods.

6. The intramedullary nail according to claim 5, characterized in that said threaded plug is conical.

7. The intramedullary nail according to claim 4, characterized in that said second pair of wire rods comprises wire rods whose point is angled with respect to the central portion of said wire rod and is rotated through 90° with respect thereto.

8. The intramedullary nail according to claim 3, characterized in that a bush is provided arranged at the proximal region of said tube and allows the insertion of said wire rods and their guiding within said tube.

9. The intramedullary nail according to claim 3, characterized in that it comprises a tool for determining drilling points for the insertion of at least one screw for locking the proximal region of the tube to the femur or tibia.

10. The intramedullary nail according to claim 9, characterized in that said tool comprises a point which is suitable to be inserted in said tube and to which a template portion corresponds externally, said template portion allowing to determine a bone drilling point for the passage of said at least one locking screw within a pair of holes formed in said tube at the proximal region.
